# EUROPÄISCHE PATENTSCHRIFT

(11) **EP 3 405 138 B1**
(45) Veröffentlichungstag und Bekanntmachung des Hinweises auf die Patenterteilung: **18.12.2019**
(21) Anmeldenummer: 17703655.5
(22) Anmeldetag: 12.01.2017
(51) Int. Cl.: A61F 2/07, A61F 2/915

(54) **DOPPELSTENT**
DOUBLE STENT
STENT DOUBLE

(30) Priorität: 19.01.2016 DE 102016100774
(43) Veröffentlichungstag der Anmeldung: 28.11.2018
(73) Patentinhaber: Bentley InnoMed GmbH, 72379 Hechingen (DE)
(72) Erfinder: BREGULLA, Rainer, 72336 Balingen (DE); OBRADOVIC, Milisav, 79539 Lörrach (DE)
(74) Vertreter: Schneiders & Behrendt PartmbB Rechtsanwälte - Patentanwälte
(86) Internationale Anmeldenummer: PCT/EP2017/050574
(87) Internationale Veröffentlichungsnummer: WO 2017/125312

(56) Entgegenhaltungen:
- EP-A1- 0 878 173
- WO-A2-2012/084202
- DE-U1- 29 623 983

## Beschreibung

Die Erfindung betrifft einen Doppelstent mit 2 koaxial angeordneten Stents, wobei zwischen einem ersten inneren Stent und einem zweiten äußeren Stent eine erste Membran angeordnet ist. Der Doppelstent dient insbesondere als Stentgraft zur Überbrückung von vaskulären Fehlbildungen, etwa Aneurysmen und Shunts, aber auch zur Verstärkung von labilen, brüchigen oder thrombotischen Gefäßwänden. Er wird ferner eingesetzt als Überbrückung bei Abzweigungen aus gestenteten Gefäßen.

Stentgrafts zur Überbrückung von vaskulären Fehlbildungen sind in einer Vielzahl von Formen bekannt. In der Regel bestehen sie aus einem Stent, der mit einer Membran ganz oder teilweise überzogen ist. Die Membran schließt die vaskuläre Fehlbildung gegen das Gefäß ab, der Stent hält das Gefäß offen und sorgt dafür, dass die Membran eng an der Gefäßwand anliegt.

Ein Problem bei Stentgrafts ist die Verankerung der Membran am Stent. Hierzu wurden Doppelstents entwickelt, bei denen die Membran zwischen einem äußeren und einem inneren Stent gehalten wird. Bei der Expansion eines solchen Doppelstents nimmt die Membran an der radialen Erweiterung teil, bleibt aber zwischen den beiden Stents eingeklemmt.

Ein solcher Doppelstent ist beispielsweise aus der DE 197 20 115 A1 bekannt. Der dort beschriebene Stent hat sich an und für sich bewährt, ist aber in zweierlei Hinsicht verbesserungsfähig.

Zum einen ergeben sich häufig Probleme mit der Dichtigkeit, da die Membran nicht unmittelbar an der Gefäßwand anliegt und/oder bei der Expansion des Doppelstents beschädigt wird. In beiden Fällen wird der Doppelstent den an ihn gestellten Anforderungen, nämlich der Abschottung beispielsweise einer vaskulären Fehlbildung, nicht gerecht.

Zum anderen kann es bei der Expansion des Doppelstents dazu kommen, dass der Verbund aus zwei Stents und einer Membran an Zusammenhalt verliert, beispielsweise wenn die beiden Stents ein unterschiedliches Expansionsverhalten - etwa aufgrund örtlicher Gegebenheiten - haben.

Bei der Verwendung normaler Stentgrafts reicht in zahlreichen Fällen die Radialkraft eines einzelnen Stents, sei er ballonexpandierbar oder selbst expandierend, nicht aus, um die Membran sicher zu verankern und/oder eine zuverlässige und dauerhafte Überbrückung oder Gefäßerweiterung sicherzustellen. In diesen Fällen wäre der Einsatz eines Doppelstents mit erhöhter Radialkraft sinnvoll. Dies gilt insbesondere für selbst expandierende Stents, die in der Regel eine geringere Radialkraft haben, als ballonexpandierbare Stents.

Der Erfindung liegt damit die Aufgabe zu Grunde, einen Doppelstent bereitzustellen, der zum einen den Anforderungen an die Dichtigkeit und Zuverlässigkeit genügt und zum anderen den notwendigen Zusammenhalt gewährleistet. Darüber hinaus soll der Stent eine hohe Radialkraft aufweisen.

Diese Aufgabe wird mit einem Doppelstent der eingangs genannten Art gelöst, bei dem auf dem zweiten äußeren Stent eine zweite Membran angeordnet ist, wobei die Membranenden der ersten und der zweiten Membran an den Enden der Stents zusammengefasst sind, auf die Innenseite des ersten Stents umgeschlagen sind und unter Federzungen des ersten Stents festgeklemmt sind.

DE 296 23 983 U1 beschreibt ein intraluminales Implantat, das ein Polytetrafluorethylen (PTFE) Material enthält, das sich zwischen zwei in bezug auf den Aufbau ähnlichen Stützmaterialien, wie jene Materialien, die Stents umfassen, befindet.

Der erfindungsgemäße Doppelstent weist nicht nur einen inneren und einen äußeren Stent auf sondern auch eine innere und äußere Membran. Dabei ergänzen sich die beiden Stents hinsichtlich der Radialkraft und die beiden Membranen hinsichtlich der Dichtigkeit. Die äußere zweite Membran dient als Schutz und Ergänzung der inneren ersten Membran; wenn die innere Membran bei der Expansion beschädigt wird, beispielsweise einreißt; ist die äußere Membran in der Lage, diesen Defekt auszugleichen und umgekehrt. Des Weiteren hält die äußere Membran das Konstrukt zusammen, wobei Verankerung der Enden der äußeren Membran - zusammen mit den Enden der inneren Membran - auf der Innenseite des inneren Stent zu diesem Zusammenhalt beiträgt.

Für die erfindungsgemäß eingesetzten Stents kommen übliche Stentdesigns infrage, wie sie vielfach bei ballonexpandierbaren und selbst expandierenden Stents entwickelt wurden. Für ballonexpandierbare Stents kommen übliche Materialien in Frage, beispielsweise Stahllegierungen für den medizinischen Gebrauch, Kobalt-Chrom-Legierungen und dergleichen. Für selbst expandierende Stents kommen insbesondere Materialien mit einem Formgedächtnis infrage, etwa Nickel-Titan-Legierungen.

Die Stents können geflochten sein, werden aber in der Regel aus einem Rohr mit geeignetem Durchmesser mithilfe eines Laserstrahls geschnitten. Sie verfügen über eine Maschenstruktur
Die Stents können beispielsweise eine Maschenstruktur haben, wie sie von einander kreuzenden Stegen gebildet wird. Bevorzugt sind Stents, die aus einer Mehrzahl von mäandrierenden Ringsegmenten gebildet werden, wobei die Ringsegmente durch Verbindungsstege mit benachbarten Ringsegmenten verbunden sind. Auch in diesem Fall entstehen Maschen, deren Größe durch die Häufigkeit der Verbindungsstege zwischen zwei benachbarten Ringsegmenten bestimmt ist. Eine solche Stentstruktur ist geeignet, die bei der Expansion auftretende Längenreduktion zumindest teilweise auszugleichen, je nach Anordnung und Form der Verbindungsstege.

Die am inneren ersten Stents vorhandenen Federzungen können eine Vielzahl von Formen haben. Die Federzungen weisen dabei stentauswärts, d.h., sie weisen zum Stentrand. Solche Federzungen können beispielsweise auswärts weisende Schlingen der Ringsegmente sein, wobei die Folienenden zwischen den Schlingen und vom gleichen Ringsegmente ausgehenden Verbindungsstegen festgeklemmt werden.

Der innere Stent kann auch speziell ausgebildete Federzungen, beispielsweise in Form von Blindstegen, die in den Schlingen oder an auswärtsweisenden Schlingenbögen angeordnet sind und keine Verbindungen zu benachbarten Ringsegmenten herstellen. Federzungen können auch als Einschnitte in den Schlingenbögen vorliegen, dergestalt, dass die Schlingenbögen gespalten sind und die Membranenden nach Art einer Büroklammer festhalten.

Das Festklemmen der Membranenden auf der Innenseite des inneren Stent führt zu einer zuverlässigen Verankerung der beiden Membranen und stärkt den Verbund aus innerem Stent, innerer Membran, äußerem Stent und äußerer Membran.

Die Federzungen des inneren Stents weisen immer stentauswärts. Sie befinden sich in der Regel im Randbereich des inneren Stents, dort vorzugsweise an dem zum randständigen Ringsegment benachbarten Ringsegment.

Für die Membranen kann jedes dafür geeignete biologische oder künstliche Material verwandt werden. In der Regel bestehen die Membranen aus einem Kunststoff, vorzugsweise einem Kunststoffschlauch, der über den jeweiligen Stent gezogen ist. Ein geeignetes Material ist beispielsweise Polytetrafluorethylen, PTFE, insbesondere ePTFE, dass die benötigte Elastizität für die Expansion aufweist. Andere medizinisch geeignete Kunststoffe, etwa Polyester, Polyolefine, Polyurethane, Polyurethancarbonat und dergleichen, können ebenfalls eingesetzt werden.

Es versteht sich, dass für den inneren und den äußeren Stent unterschiedliche Designs verwandt werden können und die innere und äußere Membran aus verschiedenen Materialien gefertigt sein können.

Die Verwendung von zwei Stents und zwei Membranen führt naturgemäß zu einer relativ hohen Wandstärke des Konstrukts, was die Manövrierfähigkeit im Gefäßsystem eines Patienten einschränkt. Dem kann dadurch begegnet werden, dass die Wandstärke der zum Schneiden der Stents verwandten Rohre gering gewählt wird, beispielsweise im Bereich von 0,05 bis 0,50 mm, vorzugsweise 0,10 bis 0,20 mm und insbesondere etwa 0,15 mm. Auch die Stegbreite kann vermindert werden auf beispielsweise 0,05 bis 0,50 mm, vorzugsweise 0,10 bis 0,20 mm und insbesondere etwa 0,15 mm. Durch die Verwendung von zwei Stents wird dennoch eine hohe Radialkraft erreicht.

Es ist ferner bevorzugt, den äußeren Stent mit kleineren Maschen zu versehen als den inneren Stent. Hierdurch wird bei der Expansion eine Pressspannung erzeugt, die sich vorteilhaft auf die Radialkraft und den Zusammenhalt des Konstrukts auswirkt. Erzielt wird eine hohe Festigkeit und Dauerhaftigkeit des Konstrukts.

Der erfindungsgemäße Doppelstent ist insbesondere geeignet, in Abzweige aus gestenteten Gefäßen eingesetzt zu werden und den sich dabei zwischen gestentetem Gefäß und Abzweigung bildenden Raum zu überbrücken.

Die Erfindung wird durch die beiliegenden Abbildungen näher erläutert, die bevorzugte Ausführungsformen der Erfindung zeigen. Es versteht sich, dass in den Abbildungen gezeigten Merkmale jeweils für sich Teil der Erfindung sind und nicht nur im Zusammenhang mit anderen Merkmalen der Abbildung zu verstehen sind. Es zeigen:
- Figur 1: schematisch den Aufbau eines erfindungsgemäßen Stents;
- Figur 2: eine Variante des zum Einsatz kommenden Klemmprinzips der Membranen; und
- Figur 3: eine weitere Variante des Klemmprinzips.

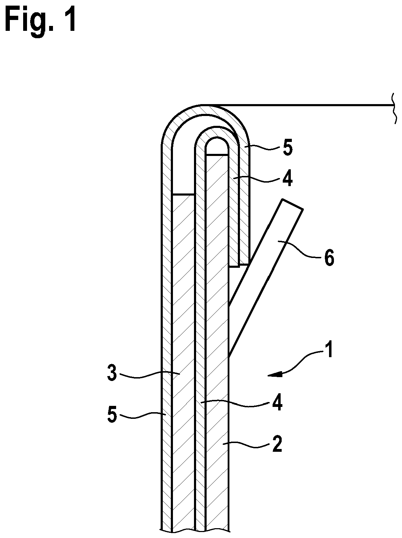

Der Doppelstent 1 gemäß Figur 1 besteht aus einem ersten inneren Stent 2 und einem zweiten äußeren Stent 3, die koaxial zueinander angeordnet sind. Der äußere Stent 3 ist um ein geringes kürzer als der innere Stent 2. Der Doppelstent ist im nicht expandierten Zustand dargestellt. Zwischen dem inneren Stent 2 und dem äußeren Stent 3 befindet sich eine erste innere Membran 4, auf dem äußeren Stent 3 eine zweite äußere Membran 5. Beide Membranen bestehen aus ePTFE.

Die innere Membran 4 und die äußere Membran 5 werden an ihren beiden Enden jeweils zusammengefasst und um den Rand der beiden Stents nach innen in den Hohlraum des inneren Stents 2 umgeschlagen. Schematisch dargestellt ist eine von mehreren Federzungen 6, die nach innen aufgebogen ist und unter die die Membranenden ragen. Die Federzungen 6 werden zur Festlegung der Membranenden nach außen zurückgebogen, so dass die Membranenden unter ihnen festgeklemmt sind.

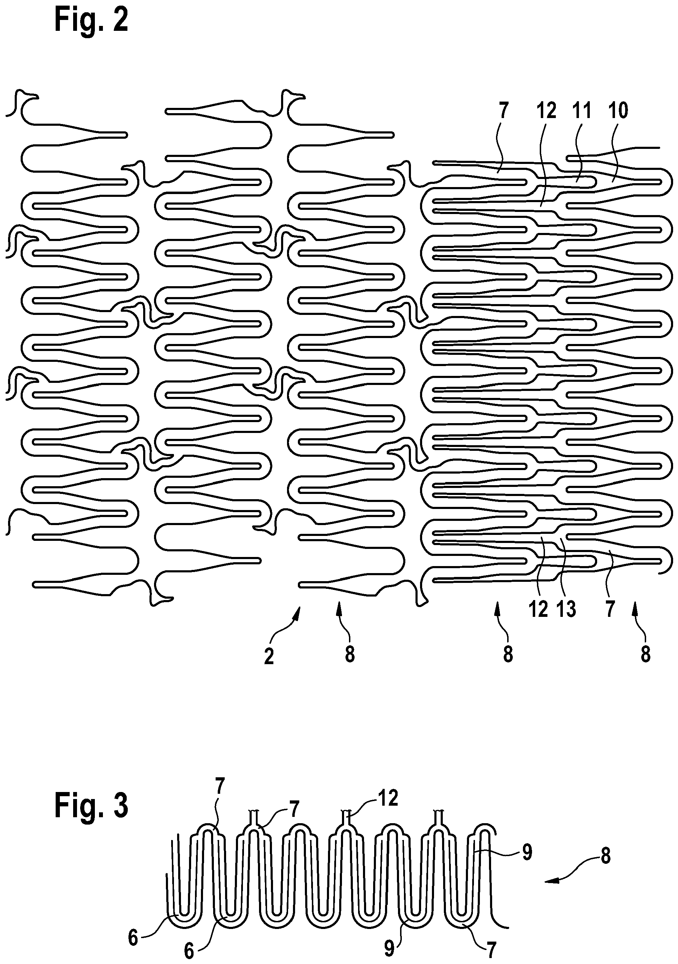

Figur 2 zeigt ein Stentdesign für den inneren Stent 2, bei dem in Eintiefungen 10 eines Ringsegments 8 Blindstege 11 hineinragen, unter die eine Membran 4/5 geschoben werden kann, so dass sie zwischen den Schlingen 7 des Ringsegments 8 und den Blindstegen 11 festgehalten wird. Benachbarte Ringsegmente 8 sind durch Verbindungsstege 12, die an den Umkehrpunkten 13 ansetzen miteinander verbunden.

Figur 3 zeigt ein Ringsegment eines Stentdesigns, bei dem im die Schlingen 7 des Ringsegments 8 Bögen 9 eingeschnitten sind, so dass senkrecht zur Stentachse bewegliche Federzungen 6 entstehen, die in der Lage sind, eine darunter geschobenen Membran 4/5 nach Art einer Büroklammer festzuhalten. Das Stentdesign ist Teil eines inneren Stents 2.

## Patentansprüche

1. Doppelstent mit zwei koaxial angeordneten Stents, wobei zwischen einem ersten inneren Stent (2) und einem zweiten äußeren Stent (3) eine erste Membran (4) angeordnet ist, **dadurch gekennzeichnet, dass** auf dem zweiten Stent (3) eine zweite Membran (5) angeordnet ist und dass die Membranenden der ersten und der zweiten Membran an den Enden der Stents zusammengefasst sind und auf die Innenseite des erstens Stents (2) umgeschlagen und unter Federzungen (6) des ersten Stents (2) festgeklemmt sind.

2. Doppelstent nach Anspruch 1, **dadurch gekennzeichnet, dass** wenigstens einer der Stents (2, 3) eine Maschenstruktur aufweist.

3. Doppelstent nach Anspruch 1 oder 2, **dadurch gekennzeichnet, dass** wenigstens einer der Stents (2, 3) eine Mehrzahl von nebeneinander angeordneten Ringsegmenten (8) mit mäandrierender Struktur aufweist, die durch Stege (12) miteinander verbunden sind.

4. Doppelstent nach einem der vorstehenden Ansprüche, **dadurch gekennzeichnet, dass** die Federzungen (6) in Maschen oder Schlingen (7) des ersten Stents (2) angeordnete Blindstege (11) sind.

5. Doppelstent nach einem der Ansprüche 1 bis 3, **dadurch gekennzeichnet, dass** die Federzungen (6) von Einschnitten (9) in Stegschlingen (7) des ersten Stents (2) gebildet sind.

6. Doppelstent nach einem der vorstehenden Ansprüche, **dadurch gekennzeichnet, dass** die Federzungen (6) stentauswärts weisen.

7. Doppelstent nach einem der vorstehenden Ansprüche, **dadurch gekennzeichnet, dass** die Federzungen (6) in den Randbereichen des ersten Stents (2) angeordnet sind.

8. Doppelstent nach Anspruch 7, **dadurch gekennzeichnet, dass** die Federzungen (6) an Ringsegmenten (8) ausgebildet sind, die benachbart zu randständigen Ringsegmenten (8) angeordnet sind.

9. Doppelstent nach einem der vorstehenden Ansprüche, **dadurch gekennzeichnet, dass** der zweite Stent (3) eine dichtere Struktur aufweist als der erste Stent (2).

10. Doppelstent nach einem der vorstehenden Ansprüche, **dadurch gekennzeichnet, dass** die Stege des ersten (2) und zweiten Stents (3) auf Lücke stehen.

11. Doppelstent nach einem der vorstehenden Ansprüche, **dadurch gekennzeichnet, dass** die erste (4) und/oder zweite Membran (5) aus einem Kunststoff besteht.

12. Doppelstent nach Anspruch 11, **dadurch gekennzeichnet, dass** die erste (4) und/oder zweite Membran (5) aus PTFE besteht.

## Claims

1. Double stent comprising two coaxially arranged stents, wherein a first membrane (4) being arranged between a first inner stent (2) and a second outer stent (3), **characterized in that** a second membrane (5) is arranged on the second stent (3) and that the membrane ends of the first and second membrane are brought together at the ends of the stents and are folded over onto the inside of the first stent (2) and clamped under flexible tongues (6) of the first stent (2).

2. Double stent according to claim 1, **characterized in that** at least one of the stents (2, 3) has a mesh structure.

3. Double stent according to claim 1 or 2, **characterized in that** at least one of the stents (2, 3) is provided with a plurality of ring segments (8) arranged side by side and having a meandering structure which are connected to one another by webs (12).

4. Double stent according to any one of the preceding claims, **characterized in that** the flexible tongues (6) are blind webs (11) arranged in meshes or loops (7) of the first stent (2).

5. Double stent according to any one of claims 1 to 3, **characterized in that** the flexible tongues (6) are formed by applying incisions (9) in the web loops (7) of the first stent (2).

6. Double stent according to any one of the preceding claims, **characterized in that** the flexible tongues (6) point to the outside of the stent.

7. Double stent according to any one of the preceding claims, **characterized in that** the flexible tongues (6) are arranged in the peripheral regions of the first stent (2).

8. Double stent according to claim 7, **characterized in that** the flexible tongues (6) are formed on ring segments (8) arranged adjacent to the peripheral ring segments (8).

9. Double stent according to any one of the preceding claims, **characterized in that** the second stent (3) has a structure that is denser than that of the first stent (2).

10. Double stent according to any one of the preceding claims, **characterized in that** the webs of the first (2) and second stent (3) are arranged staggered.

11. Double stent according to any one of the preceding claims, **characterized in that** the first (4) and/or second membrane (5) consists of plastic material.

12. Double stent according to claim 11, **characterized in that** the first (4) and/or second membrane (5) consists of PTFE.

## Revendications

1. Double stent comprenant deux stents disposés coaxialement, une première membrane (4) étant disposée entre un premier stent intérieur (2) et un deuxième stent extérieur (3), **caractérisé en ce qu'**une deuxième membrane (5) est disposée sur le deuxième stent (3) et **en ce que** les extrémités de membrane de la première et de la deuxième membrane sont réunies au niveau des extrémités des stents et sont retournées sur le côté intérieur du premier stent (2) et sont serrées fixement sous des langues de ressort (6) du premier stent (2).

2. Double stent selon la revendication 1, **caractérisé en ce qu'**au moins l'un des stents (2, 3) présente une structure à mailles.

3. Double stent selon la revendication 1 ou 2, **caractérisé en ce qu'**au moins l'un des stents (2, 3) présente une pluralité de segments annulaires (8) disposés les uns à côté des autres, avec une structure en méandres, lesquels sont connectés les uns aux autres par des nervures (12).

4. Double stent selon l'une quelconque des revendications précédentes, **caractérisé en ce que** les langues de ressort (6) sont des nervures aveugles (11) disposées dans des mailles ou des boucles (7) du premier stent (2).

5. Double stent selon l'une quelconque des revendications 1 à 3, **caractérisé en ce que** les langues de ressort (6) sont formées par des entailles (9) dans des boucles de nervures (7) du premier stent (2).

6. Double stent selon l'une quelconque des revendications précédentes, **caractérisé en ce que** les langues de ressort (6) sont orientées vers l'extérieur du stent.

7. Double stent selon l'une quelconque des revendications précédentes, **caractérisé en ce que** les langues de ressort (6) sont disposées dans les régions de bord du premier stent (2).

8. Double stent selon la revendication 7, **caractérisé en ce que** les langues de ressort (6) sont réalisées au niveau de segments annulaires (8) qui sont disposés à côté de segments annulaires au niveau des bords (8).

9. Double stent selon l'une quelconque des revendications précédentes, **caractérisé en ce que** le deuxième stent (3) présente une structure plus épaisse que le premier stent (2).

10. Double stent selon l'une quelconque des revendications précédentes, **caractérisé en ce que** les nervures du premier (2) et du deuxième (3) stent sont situées sur des espaces vides.

11. Double stent selon l'une quelconque des revendications précédentes, **caractérisé en ce que** la première (4) et/ou la deuxième (5) membrane se composent d'un plastique.

12. Double stent selon la revendication 11, **caractérisé en ce que** la première (4) et/ou la deuxième (5) membrane se composent de PTFE.
